# EUROPEAN PATENT APPLICATION

(11) **EP 2 145 620 A2**
(43) Date of publication of application: **20.01.2010**
(21) Application number: 09173892.2
(22) Date of filing: 25.06.2004
(51) Int. Cl.: A61K 31/437, A61K 31/445, A61K 31/343, A61K 31/4439, A61K 31/404, A61K 31/15, A61P 25/18

(54) **Gaboxadol for treating depression and other affective disorders**

(30) Priority: 25.06.2003 DK 200300956; 25.06.2003 US 483019 P; 07.01.2004 US 535123 P; 07.01.2004 DK 200400016
(62) Divisional of application: 04738956.4
(71) Applicant: H. LUNDBECK A/S, 2500 Valby (DK)
(72) Inventor: Sanchez, Connie, West Milford, NJ 07480 (US); Ebert, Bjarke, 3520, Farum (DK)

(57) **Abstract**

The present invention relates to use of gaboxadol for preparing a pharmaceutical composition for treating depression. Moreover, it relates to the use of gaboxadol for the preparation of a pharmaceutical composition to be used in combination with a serotonin reuptake inhibitor or any other compound, which causes an elevation in the level of extracellular serotonin.

## Description

### Field of invention

The present invention relates to the use of gaboxadol for the preparation of medicaments useful for the treatment of depression. The present invention also relates to the use of a combination of gaboxadol and a serotonin reuptake inhibitor (SRI), or any other compound, which causes an elevation in the level of extracellular serotonin, for the treatment of depression and other affective disorders.

### Background of the Invention

Gaboxadol (THIP), described in EP patent 0000338 B1 and in EP Patent 0840601 B1, have shown great potential in the treatment of sleep disorders.

Selective serotonin reuptake inhibitors (hereinafter referred to as SSRIs) have become first choice therapeutics in the treatment of depression, certain forms of anxiety and social phobias, because they are effective, well tolerated and have a favourable safety profile compared to the classic tricyclic antidepressants.

However, clinical studies on depression and anxiety disorders indicate that non-response to SSRIs is substantial, up to 30%. Another, often neglected, factor in antidepressant treatment is compliance, which has a rather profound effect on the patient's motivation to continue pharmacotherapy.

### Description of the Invention

According to the present invention a pharmaceutical composition for the treatment of depression is provided.

### Gaboxadol has the general formula

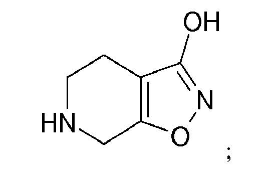

and throughout the description "gaboxadol" is intended to include any form of the compound, such as the base (zwitter ion), pharmaceutically acceptable salts, e.g. pharmaceutically acceptable acid addition salts, hydrates or solvates of the base or salt, as well as anhydrates, and also amorphous, or crystalline forms.

More specifically, the present invention relates to the use of gaboxadol having the general formula 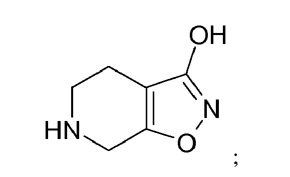 for the preparation of a pharmaceutical composition for the treatment of depression.

In a further aspect, the present invention relates to a method for the treatment of depression comprising administering to an individual in need thereof a pharmaceutically acceptable amount of gaboxadol. Such individual is preferably a human, such as male or female human, child, adult or elderly.

According to the invention, gaboxadol may be used as the base (the zwitter ion) or as a pharmaceutically acceptable acid addition salt thereof or as an anhydrate or hydrate of such salt or base. The salts of the compound used in the invention are salts formed with non-toxic organic or inorganic acids. Exemplary of such organic salts are those with maleic, fumaric, benzoic, ascorbic, succinic, oxalic, bis-methylenesalicylic, methanesulfonic, ethane-disulfonic, acetic, propionic, tartaric, salicylic, citric, gluconic, lactic, malic, mandelic, cinnamic, citraconic, aspartic, stearic, palmitic, itaconic, glycolic, p-amino-benzoic, glutamic, benzene sulfonic and theophylline acetic acids, as well as the 8-halotheophyllines, for example 8-bromo-theophylline. Exemplary of such inorganic salts are those with hydrochloric, hydrobromic, sulfuric, sulfamic, phosphoric and nitric acids. Gaboxadol may also be used as the zwitter ion, e.g. the mono hydrate thereof.

The acid addition salts according to the invention may be obtained by treatment of gaboxadol with the acid in an inert solvent followed by precipitation, isolation and optionally re-crystallisation by known methods and if desired micronisation of the crystalline product by wet or dry milling or another convenient process, or preparation of particles from a solvent-emulsification process. Suitable methods are described in EP patent 0000338.

Precipitation of the salt is typically carried out in an inert solvent, e.g. an inert polar solvent such as an alcohol (e.g. ethanol, 2-propanol and n-propanol), but water or mixtures of water and inert solvent may also be used.

In an embodiment, gaboxadol is used in the form of an acid addition salt, or a zwitter ion hydrate or zwitter ion anhydrate. In a further embodiment, gaboxadol is used in the form of the pharmaceutically acceptable acid addition salt selected from the hydrochloride or hydrobromide salt, or in the form of the zwitter ion monohydrate. Most conveniently, gaboxadol is in a crystalline form.

According to the invention, gaboxadol may be administered in any suitable way e.g. orally or parenterally, and it may be presented in any suitable form for such administration, e.g. in the form of tablets, capsules, powders, syrups or solutions or dispersions for injection. Preferably, and in accordance with the purpose of the present invention, gaboxadol is administered in the form of a solid pharmaceutical entity, suitably as a tablet or a capsule or in the form of a suspension, solution or dispersion for injection.

Methods for the preparation of solid pharmaceutical preparations are well known in the art. Tablets may thus be prepared by mixing the active ingredients with ordinary adjuvants and/or diluents and subsequently compressing the mixture in a convenient tabletting machine. Examples of adjuvants or diluents comprise: corn starch, lactose, talcum, magnesium stearate, gelatine, lactose, gums, and the like. Any other adjuvant or additive such as colourings, aroma, preservatives, etc. may also be used provided that they are compatible with the active ingredients.

A suitable formulation of gaboxadol is described in WO 02/094225 filed May 17, 2002. Without limiting the invention in any way, it is intended that any one of the aspects or embodiments of this patent application is suitable embodiments of the medicament or pharmaceutical compositions herein.

Gaboxadol is typically administered as an oral dose form, such as a solid oral dose form, typically tablets or capsules, or as a liquid oral dose form. Gaboxadol is most conveniently administered orally in unit dosage forms such as tablets or capsules, containing the active ingredient in an amount from about 0.1 to about 150 mg/day, such as from about 0.1 to about 100 mg/day, typically from about 0.5 to about 50 mg/day, preferably from about 2.5 to about 20 mg/day, e.g. from about 5 to about 15 mg/day. The amount of gaboxadol is calculated based on the free base form.

Gaboxadol may be administered as monotherapy or as combination therapy with other drugs. In a particular aspect, gaboxadol may be combined with a serotonin reuptake inhibitor as described below.

### The combination of gaboxadol with a serotonin reuptake inhibitor

In addition, gaboxadol may be used to augment and provide faster onset of the therapeutic effect of serotonin reuptake inhibitors, in particular citalopram and escitalopram.

It has now surprisingly been found that gaboxadol may be used to augment and provide faster onset of the therapeutic effect of serotonin reuptake inhibitors.

In one aspect, the invention relates to use of gaboxadol for the preparation of a pharmaceutical composition to be used in combination with a serotonin reuptake inhibitor or any other compound, which causes an elevation in the level of extracellular serotonin.

In another aspect, the invention relates to use of gaboxadol for the preparation of a pharmaceutical composition useful for augmenting and/or providing faster onset of the therapeutic effect of a serotonin reuptake inhibitor or any other compound, which causes an elevation in the level of extracellular serotonin.

In a further aspect, the invention relates to a pharmaceutical composition or kit comprising gaboxadol and a compound, which is a serotonin reuptake inhibitor, or any other compound which causes an elevation in the level of extracellular serotonin, and optionally pharmaceutically acceptable carriers or diluents.

In a further aspect, the invention relates to a method for the treatment of diseases or disorders responsive to a serotonin reuptake inhibitor or any other compound which causes an elevation in the level of extracellular serotonin, comprising administering gaboxadol and a serotonin reuptake inhibitor, or a compound which causes an elevation in the level extracellular serotonin, to an individual in need thereof.

In a further aspect, the invention relates to use of gaboxadol and a compound, which is a serotonin reuptake inhibitor, or any other compound which causes an elevation in the level of extracellular serotonin, for the preparation of a pharmaceutical composition for the treatment of diseases or disorders responsive to the therapeutic effect of a serotonin reuptake inhibitor or any other compound causing an elevation in the level of extracellular serotonin.

In a further aspect, the invention relates to use of gaboxadol for the preparation of a pharmaceutical composition for the treatment of an individual to be treated with or undergoing treatment with the serotonin reuptake inhibitor, or any other compound which causes an elevation in the level of extracellular serotonin, wherein said individual suffers from diseases or disorders responsive to the therapeutic effect of a serotonin reuptake inhibitor or any other compound causing an elevation in the level of extracellular serotonin.

In a further aspect, the invention relates to use of gaboxadol and a compound, which is a serotonin reuptake inhibitor, or any other compound which causes an elevation in the level of extracellular serotonin, for the preparation of a kit for the treatment of diseases or disorders responsive to the therapeutic effect of a serotonin reuptake inhibitor or any other compound causing an elevation in the level of extracellular serotonin.

In a further aspect, the invention relates to a method for augmenting and/or providing faster onset of the therapeutic effect of a serotonin reuptake inhibitor, or any other compound which causes an elevation in the level of extracellular serotonin, comprising administering gaboxadol to an individual to be treated with or undergoing treatment with the serotonin reuptake inhibitor, or any other compound which causes an elevation in the level of extracellular serotonin. Such individual is preferably a human, such as male or female human, child, adult or elderly.

In an embodiment, the serotonin reuptake inhibitor or the compound which causes an elevation in the level of extracellular serotonin is used in the treatment of depression, anxiety disorders and other affective disorders, eating disorders such as bulimia, anorexia and obesity, phobias, dysthymia, premenstrual syndrome, cognitive disorders, impulse control disorders, attention deficit hyperactivity disorder and drug abuse, in particular depression.

In a further embodiment, the serotonin reuptake inhibitor or the compound which causes an elevation in the level of extracellular serotonin is used in the treatment of anxiety disorders including general anxiety disorder, panic anxiety, obsessive compulsive disorder, acute stress disorder, post trauma stress disorder or social anxiety disorder.

In a further embodiment, the serotonin reuptake inhibitor is selected from citalopram, escitalopram, fluoxetine, sertraline, paroxetine, fluvoxamine, venlafaxine, duloxetine, dapoxetine, nefazodone, imipramin, femoxetine and clomipramine or a pharmaceutically acceptable salt of any of these compounds. Just to clarify, each of the serotonin reuptake inhibitors specified above is intended to be an individual embodiment. Accordingly, each of them and the use thereof may be claimed individually.

In a further embodiment, the serotonin reuptake inhibitor is a selective serotonin reuptake inhibitor (SSRI).

In a further embodiment, the pharmaceutical composition or kit prepared is adapted for simultaneous administration of the active ingredients. In an embodiment, the active ingredients are contained in the same unit dosage form.

In a further embodiment, the pharmaceutical composition or kit prepared is adapted for sequential administration of the active ingredients. In an embodiment, the active ingredients are contained in discrete unit dosage forms.

In a further aspect, the present invention relates to the use of gaboxadol for the preparation of a pharmaceutical composition to be used in combination with a serotonin reuptake inhibitor or any other compound which causes an elevation in the level of extracellular serotonin.

In particular, the present invention relates to the use of gaboxadol for the preparation of a pharmaceutical composition useful for augmenting and/or providing faster onset of the therapeutic effect of a serotonin reuptake inhibitor or any other compound which causes an elevation in the level of extracellular serotonin.

More particularly, the present invention relates to the use as above, of gaboxadol, for the treatment of depression, anxiety disorders and other affective disorders, such as generalized anxiety disorder, panic anxiety, obsessive compulsive disorder, acute stress disorder, post traumatic stress disorder and social anxiety disorder eating disorders such as bulimia, anorexia and obesity, phobias, dysthymia, premenstrual syndrome, cognitive disorders, impulse control disorders, attention deficit hyperactivity disorder and drug abuse, in particular depression with a serotonin reuptake inhibitor or any other compound which causes an elevation in the level of extracellular serotonin.

The anxiety disorders mentioned above include general anxiety disorder, panic anxiety, obsessive compulsive disorder, acute stress disorder, post trauma stress disorder or social anxiety disorder.

As used herein, the term augmenting covers improving the therapeutic effect and/or potentiating the therapeutic effect of an SRI or a compound which causes an elevation in the level of extracellular 5-HT.

In a further embodiment, the invention relates to the use of gaboxadol and a compound, which is a serotonin reuptake inhibitor, or a compound, which causes an elevation in the level of extracellular serotonin, for the preparation of a pharmaceutical composition for the treatment of diseases or disorders responsive to the therapeutic effect of a serotonin reuptake inhibitor, or any other compound, which causes an elevation in the level of extracellular serotonin.

In a further embodiment, the invention relates to the use of gaboxadol and a compound, which is a serotonin reuptake inhibitor, or a compound, which causes an elevation in the level of extracellular serotonin, for the preparation of a kit-of-parts (kit) for the treatment of diseases or disorders responsive to the therapeutic effect of a serotonin reuptake inhibitor, or any other compound, which causes an elevation in the level of extracellular serotonin.

The diseases responsive to a serotonin reuptake inhibitor include depression, anxiety disorders and other affective disorders, eating disorders such as bulimia, anorexia and obesity, phobias, dysthymia, premenstrual syndrome, cognitive disorders, impulse control disorders, attention deficit hyperactivity disorder and drug abuse, in particular depression.

The term anxiety disorders is as defined above.

In one embodiment, the present invention relates to the use of gaboxadol for the preparation of a pharmaceutical composition as above, which is adapted for simultaneous administration of the active ingredients. In particular, such pharmaceutical compositions may contain the active ingredients within the same unit dosage form, e.g. in the same tablet or capsule. Such unit dosage forms may contain the active ingredients as a homogenous mixture or in separate compartments of the unit dosage form.

In another embodiment, the present invention relates to the use of gaboxadol for the preparation of a pharmaceutical composition or kit as above, which is adapted for sequential administration of the active ingredients. In particular, such pharmaceutical compositions may contain the active ingredients in discrete unit dosage forms, e.g. discrete tablets or capsules containing either of the active ingredients. These discrete unit dosage forms may be contained in the same container or package, e.g. a blister pack.

As used herein the term kit means a pharmaceutical composition containing each of the active ingredients, but in discrete unit dosage forms.

The invention also relates to a pharmaceutical composition or kit comprising gaboxadol and a compound, which is a serotonin reuptake inhibitor, or any other compound which causes an elevation in extracellular 5-HT, and optionally pharmaceutically acceptable carriers or diluents.

The pharmaceutical composition or kit of the invention may be adapted for simultaneous administration of the active ingredients or for sequential administration of the active ingredients, as described above.

Finally, the present invention relates to a method for the treatment of diseases or disorders responsive to a serotonin reuptake inhibitor or any other compound which causes an elevation in the level of extracellular serotonin, comprising administering gaboxadol and a serotonin reuptake inhibitor, or a compound which causes an elevation in the level extracellular serotonin, to an individual in need thereof.

In particular, the present invention relates to a method for augmenting and/or providing faster onset of the therapeutic effect of a serotonin reuptake inhibitor or any other compound which causes an elevation in the level extracellular serotonin, comprising administering gaboxadol to an individual to be treated with or undergoing treatment with the serotonin reuptake inhibitor or any other compound which causes an elevation in the level of extracellular serotonin.

The individuals, which may benefit from treatment with a combination as above, may suffer from depression, anxiety disorders and other affective disorders, eating disorders such as bulimia, anorexia and obesity, phobias, premenstrual syndrome, dysthymia, cognitive disorders, impulse control disorders, attention deficit hyperactivity disorder and drug abuse, in particular depression.

As mentioned above, anxiety disorder includes general anxiety disorder, panic anxiety, obsessive compulsive disorder, acute stress disorder, post trauma stress disorder or social anxiety disorder.

Gaboxadol and the serotonin reuptake inhibitor may be administered simultaneously as described above.

Alternatively, the active ingredients may be administered sequentially, e.g. in two discrete unit dosage forms as described above.

It has now, surprisingly, been found that co-administration of gaboxadol and a serotonin reuptake inhibitor produces a significant increased response in an animal model predictive of antidepressant effect, the mouse forced swim test, compared to the administration of the serotonin reuptake inhibitor alone.

As mentioned above, serotonin reuptake inhibitors show delayed onset of action. Even in responders to SSRIs, several weeks of treatment are necessary to achieve a relief in symptoms. Gaboxadol may provide fast onset of therapeutic effect of serotonin reuptake inhibitors.

The use of a combination of gaboxadol and a serotonin reuptake inhibitor may greatly reduce the amount of serotonin reuptake inhibitor necessary to treat depression and other affective disorders and may thus reduce the side effects caused by the serotonin reuptake inhibitor. In particular, the combination of a reduced amount of SRI and gaboxadol may reduce the risk of SSRI-induced sexual dysfunction and sleep disturbances.

Co-administration of gaboxadol and a serotonin reuptake inhibitor may also be useful for the treatment of refractory depression, i.e. depression, which cannot be treated appropriately by administration of a serotonin reuptake inhibitor alone. Typically, gaboxadol may be used as add-on therapy for the augmentation of the response to SRIs in patients where at least 40-60% reduction in symptoms has not been achieved during the first 6 weeks of treatment with an SRI.

Typically, gaboxadol is used in the form of an acid addition salt, or a zwitter ion hydrate or zwitter ion anhydrate. In a further embodiment, gaboxadol is used in the form of the pharmaceutically acceptable acid addition salt selected from the hydrochloride or hydrobromide salt, or in the form of the zwitter ion monohydrate. Most conveniently, gaboxadol is in a crystalline form.

Many antidepressants with serotonin reuptake inhibiting effect have been described in the literature. Any pharmacologically active compound, which primarily or partly exert its therapeutic effect via inhibition of serotonin reuptake in the central nervous system (CNS), may benefit from augmentation with gaboxadol.

The following list contains a number of serotonin reuptake inhibitors which may benefit from augmentation with gaboxadol: citalopram, escitalopram, fluoxetine, R-fluoxetine, sertraline, paroxetine, fluvoxamine, venlafaxine, duloxetine, dapoxetine, nefazodone, imipramine, imipramine N-oxide, desipramine, pirandamine, dazepinil, nefopam, befuraline, fezolamine, femoxetine, clomipramine, cianoimipramine, litoxetine, cericlamine, seproxetine, WY 27587, WY 27866, imeldine, ifoxetine, tiflucarbine, viqualine, milnacipran, bazinaprine, YM 922, S 33005, F 98214-TA, OPC 14523, alaproclate, cyanodothepine, trimipramine, quinupramine, dothiepin, amoxapine, nitroxazepine, McN 5652, McN 5707, Ol 77, Org 6582, Org 6997, Org 6906, amitriptyline, amitriptyline N-oxide, nortriptyline, CL 255.663, pirlindole, indatraline, LY 113.821, LY 214.281, CGP 6085 A, RU 25.591, napamezole, diclofensine, trazodone, EMD 68.843, BMY 42.569, NS 2389, sercloremine, nitroquipazine, ademethionine, sibutramine, clovoxamine, desmethylsubitramine, didesmethylsubitramine, clovoxamine vilazodone,
N-[( 1-[(6-Fluoro-2-naphthalenyl)methyl]- 4-piperidinyl]amino]carbonyl]-3-pyridine carboxamide, [trans-6-(2-chlorophenyl)-1,2,3,5,6,10b-hexahydropyrrolo- (2,1-a)isoquinoline] (McN 5707),
(dl-4-exo-amino-8-chloro-benzo-(b)-bicyclo[3.3.1]nona-2-6 alpha(10 alpha)-diene hydrochloride)(Org 6997),
(dl)-(5 alpha,8 alpha,9 alpha)-5,8,9,10-Tetrahydro-5,9-methanobenzocycloocten-8-amine hydrochloride (Org 6906),
-[2-[4-(6-fluoro-1H-indol-3-yl)-3,6-dihydro-1(2H)-pyridinyl]ethyl]-3-isopropyl-6-(methylsulphonyl)-3,4-dihydro-1H-2,1,3-benzothiadiazine-2,2-dioxide (LY393558), [4-(5,6-dimethyl-2-benzofuranyl)-piperidine] (CGP 6085),
dimethyl-[5-(4-nitro-phenoxy)-6,7,8,9-tetrahydro-5H-benzocyclohepten-7-yl]-amine (RU 25.591),

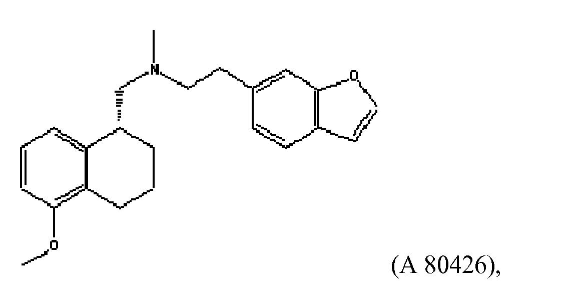

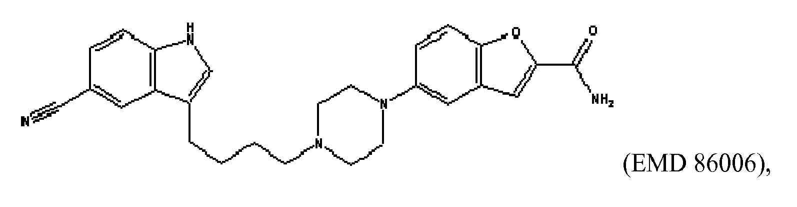

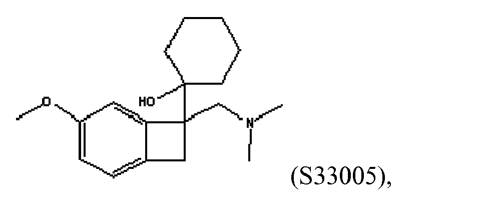

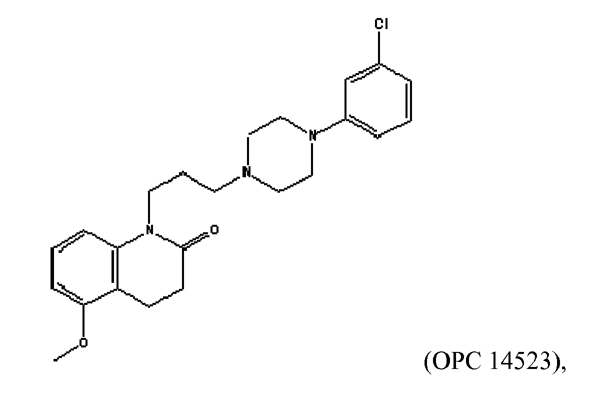

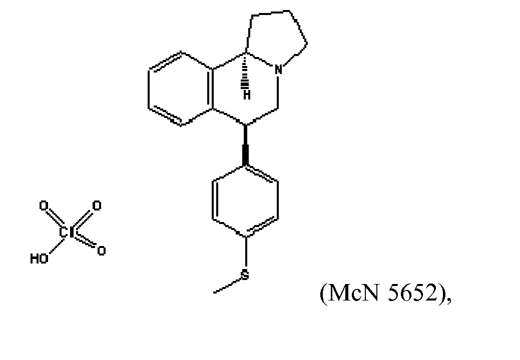

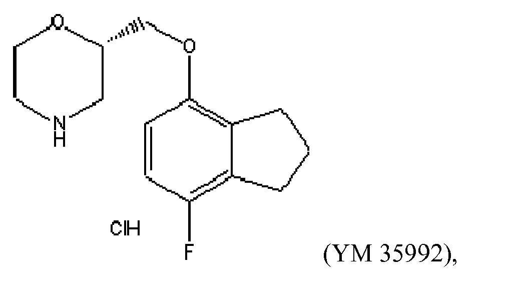

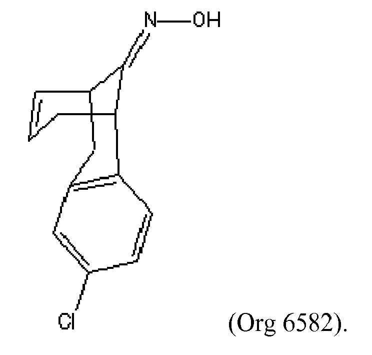

The compounds mentioned above may be used in the form of the base or a pharmaceutically acceptable salt, such as acid addition salt, thereof. Each of the serotonin reuptake inhibitors specified above is intended to be an individual embodiment. Accordingly, each of them and the use thereof may be claimed individually.

Other therapeutic compounds, which may benefit from augmentation with gaboxadol, include compounds which causes an elevation in the extracellular level of 5-HT in the synaptic cleft, although they are not serotonin reuptake inhibitors. One such compound is tianeptine.

The above list of serotonin reuptake inhibitors and other compounds which causes an increase in the extracellular level of serotonin, may not be construed as limiting.

In an embodiment, the SRIs is selected from citalopram, escitalopram, fluoxetine, sertraline, paroxetine, fluvoxamine, duloxetine, venlafaxine, duloxetine, dapoxetine, nefazodone, imipramin, femoxetine and clomipramine. Just to clarify, each of these SRIs constitute individual embodiments, and may be the subject of individual claims.

The term selective serotonin reuptake inhibitor (SSRI) means an inhibitor of the monoamine transporters which has stronger inhibitory effect at the serotonin transporter than the dopamine and the noradrenaline transporters.

Selective serotonin reuptake inhibitors (SSRIs) are among the most preferred serotonin reuptake inhibitors used according to the present invention. Thus, in a further embodiment the SRI is selected from SSRIs, such as citalopram, escitalopram, fluoxetine, fluvoxamine, sertraline or paroxetine.

The active ingredients according to the invention, i.e. gaboxadol and the SRI or a compound causing an increase in extracellular serotonin levels, may be used in the free base form or in the form of a pharmaceutically acceptable acid addition salt thereof, the latter being obtainable by reaction of the base form with an appropriate acid.

Citalopram is preferably used in the form of the hydrobromide or as the base, escitalopram in the form of the oxalate, fluoxetine, sertraline and paroxetine in the form of the hydrochloride and fluvoxamine in the form of the maleate.

As mentioned above, the combination of gaboxadol with a serotonin reuptake inhibitor unexpectedly shows a synergistic effect on the CNS. As a consequence, combination therapy using gaboxadol and lower doses of the serotonin reuptake inhibitor than normally used in monotherapy, may be effective, and side-effects associated with the larger amounts of serotonin reuptake inhibitor used in monotherapy may be reduced or prevented altogether.

Additionally, combination therapy with gaboxadol using a normal dose of serotonin reuptake inhibitor has the advantage that an effective CNS effect may be obtained in the often large number of patients who do not respond to conventional monotherapy with SSRIs.

The amount of gaboxadol used in combination therapy may range from about 0.1 to about 150 mg/day, such as from about 0.1 to about 100 mg/day, from about 0.5 to about 50 mg/day, from about 5 to about 50 mg/day, or from about 1 to about 5 mg/day. Low amounts of gaboxadol having no significant effect on sleep disorders have shown clear and significant effect in combination therapy with a serotonin reuptake inhibitor, such as escitalopram. Low amounts are typically selected from about 0.1 to about 2.5 mg/day, such as from about 0.1 to about 2.0 mg/day, whereas higher amounts are typically selected from about 2.5 to about 150 mg/day.

Serotonin reuptake inhibitors, including the SSRIs specifically mentioned hereinabove, differ both in molecular weight and in activity. As a consequence, the amount of serotonin reuptake inhibitor used in combination therapy depends on the nature of said serotonin reuptake inhibitor. In one embodiment of the invention, the serotonin reuptake inhibitor or the compound causing an increase in the level of extracellular 5-HT, is administered at lower doses than required when the compound is used alone. In another embodiment, the serotonin reuptake inhibitor or the compound causing an increase in the level of extracellular 5-HT, is administered in normal doses.

To prepare the pharmaceutical compositions of this invention, an appropriate amount of the active ingredient(s), in salt form or base form, is combined in an intimate admixture with a pharmaceutically acceptable carrier, which can take a wide variety of forms depending on the form of preparation desired for administration. These pharmaceutical compositions are desirably in unitary dosage form suitable for administration orally, rectally, percutaneously or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed.

It is especially advantageous to formulate the aforementioned pharmaceutical compositions in dosage unit form for ease of administration and uniformity of dosage. As used in the specification and claims, unit dosage form refers to physically discrete units suitable as unitary dosages, each unit containing a predetermined quantity of active ingredient(s) calculated to produce the desired therapeutic effect, in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Gaboxadol may be administered before, during or after the administration of the serotonin reuptake inhibitor provided that the time between the administration of gaboxadol and the administration of the serotonin reuptake inhibitor is such that ingredients are allowed to act synergistically on the CNS. When simultaneous administration of gaboxadol and a serotonin reuptake inhibitor is envisaged, a composition containing both a serotonin reuptake inhibitor and gaboxadol may be particularly convenient. Alternatively, gaboxadol and the serotonin reuptake inhibitor may be administered separately in the form of suitable compositions. The compositions may be prepared as described hereinabove.

The present invention also comprises products containing gaboxadol and a serotonin reuptake inhibitor as a combination preparation for simultaneous, separate or sequential use in psychiatric drug therapy. Such products may comprise, for example, a kit comprising discrete unit dosage forms containing gaboxadol and discrete unit dosage forms containing a serotonin reuptake inhibitor, all contained in the same container or pack, e.g. a blister pack.

The above mentioned preparations for simultaneous or sequential administration may instead of a serotonin reuptake inhibitor contain another compound causing an elevation in the level of extracellular 5-HT.

### Pharmacological Test of antidepressant effect of gaboxadol

The rat chronic mild stress (CMS) model of depression has a high degree of preditive validity for antidepressant activity (Willner (1997), Psychopharmacol 134:319-329). Furthermore the procedure is an appropriate model to study onset of antidepressant action in animals. (Behavioural Pharmacology 14:465-470, 2003, Sánchez, C. et al). The principle of the model is based on the relation between stress and affective disorders. Rats exposed to chronic stress will show a reduced sensitivity to rewards (e.g. a palatable sucrose solution).

### Experimental Procedure chronic mild stress

Male Wistar rats (Gorzkowska, Warsaw) were brought into the laboratory two months before the start of the experiment. The animals were singly housed with food and water freely available, and maintained on a 12-h light/dark cycle at a temperature of 22 ± 2 °C, except as described below.

The animals were first trained to consume a 1% sucrose solution; training consisted of eight 1h baseline tests in which sucrose was presented, in the home cage, following 14h food and water deprivation; intake was measured by weighing pre-weighed bottles containing the sucrose solution, at the end of the test. Subsequently, sucrose consumption was monitored, under similar conditions, throughout the whole experiment. On the basis of their sucrose intakes in the final baseline test, the animals were divided into two matched groups. One group of animals was subjected to a chronic mild stress procedure for a period of 8 consecutive weeks. The weekly stress regime consisted of 2 periods of food or water deprivation, 45 degree cage tilt, intermittent illumination (lights on and off every 2 h), soiled cage (250 ml water in sawdust bedding), paired housing and low intensity stroboscopic illumination (150 flashes/min), and 2 periods of no stress. All stressors were 10 - 14 h of duration and were applied individually and continuously, day and night. The other groups of animals, the unstressed controls, were housed in separate rooms and had no contact with the stressed animals. The rats were deprived of food and water for the 14 h preceding each sucrose test, but otherwise food and water were freely available in the home cage. On the basis of their sucrose intake scores following 3 weeks of stress, both stressed and control animals were divided into matched subgroups, and for the subsequent five weeks they received twice daily intraperitoneal drug injections (at approximately 10.00 and 17.00). Sucrose tests were performed at 10 a.m. on a weekly basis (Tuesdays). Different groups of animals (n = 8) were administered vehicle (1 ml/kg per day) or test drug. Before test sessions drugs were administered at approximately 10.00h and the sucrose tests were carried out 24h following the previous drug injection. Stress was continued throughout the entire treatment period. Body weights were assessed a baseline and by the end of drug treatment.

### Results chronic mild stress

The testing of gaboxadol in the rat chronic mild stress model showed that the compound had a significant anti-depressant-like effect. The dose of 2.5 mg/kg per day had a clear and significant effect in this model.

### Gaboxadol in combination with escitalopram

In order to modulate the activity of systems, responsible for antidepressants activity, we have characterized the interaction between gaboxadol and escitalopram (an SSRI) in a behavioural model of serotonin reuptake inhibition, the mouse 5-HTP potentiation test, and a model that is predictive of antidepressant activity, the mouse forced swim test (cf. C. Sánchez et al, Psychopharmacology (2003), 167:353-362).

### Experimental Procedures

Male NMRI/BOM mice (18-25 g; Bomholtgaard, Denmark) were used. The mice were housed in plastic cages (35 x 30 x 12 cm), 10 in each and habituated to the animal facilities for at least a week before test. The room temperature (21 ± 2°C), relative humidity (55 ± 5%), and air exchange (16 times per h) were automatically controlled. The animals had free access to commercial food pellets and tap water before test.

### Potentiation of 5-HTP-induced behaviour.

The test procedure for studies in mice is described in detail by Hyttel et al (1992). Briefly, 30 min after s.c. administration of test compound mice were given 5-HTP (100 mg/kg, i.v.). Thereafter the animals were evaluated in their home cage during a 15-min observation period with respect to stereotypy (lateral head movements), tremor, and hind limb abduction. One point was given for each symptom being present. A total of 8-16 mice were used per dose.

### Inhibition of forced swimming-induced immobility.

A mouse that is forced to swim in a spatially constrained container will exert a characteristic immobile posture. Pretreatment with an antidepressant will counteract this effect. The test was conducted as described in detail by Sánchez and Meier (Psychopharmacol 129:197-205; 1997). Briefly, a fully automated test system with 6 swim units (2000 ml glass jars filled with 1200 ml soiled water (23 - 25°C) in which a mouse had been placed previously) was used. The assessment of immobility was performed by image analysis. Thirty minutes after drug or vehicle treatment the mouse was placed into the glass jar and left in the water for a total of 6 min. The accumulated duration of immobility was measured during the last 3 min. A total of 9-18 mice were tested per dose.

### Results

Gaboxadol (2.5 mg/kg) strongly potentiated the acute effects of escitalopram ( 0.5 to 0.025 mg/kg) in the 5-HTP potentiation test and a gaboxadol dose (2.5 mg/kg) that was not active by itself in the forced swim test potentiated the antidepressant-like effect of escitalopram (2.5 and 5 mg/kg) significantly.

## Claims

1. The use of gaboxadol for the preparation of a pharmaceutical composition to be used in combination with a serotonin reuptake inhibitor or any other compound, which causes an elevation in the level of extracellular serotonin.

2. Use of gaboxadol for the preparation of a pharmaceutical composition useful for augmenting and/or providing faster onset of the therapeutic effect of a serotonin reuptake inhibitor or any other compound, which causes an elevation in the level of extracellular serotonin.

3. Use of gaboxadol for the preparation of a pharmaceutical composition for the treatment of an individual to be treated with or undergoing treatment with a serotonin reuptake inhibitor, or any other compound which causes an elevation in the level of extracellular serotonin, wherein said individual suffers from diseases or disorders responsive to the therapeutic effect of a serotonin reuptake inhibitor or any other compound causing an elevation in the level of extracellular serotonin.

4. The use of any one of claims 1-3 wherein the serotonin reuptake inhibitor or the compound which causes an elevation in the level of extracellular serotonin is used in the treatment of depression, anxiety disorders and other affective disorders, eating disorders such as bulimia, anorexia and obesity, phobias, dysthymia, premenstrual syndrome, cognitive disorders, impulse control disorders, attention deficit hyperactivity disorder and drug abuse.

5. The use of claim 4 wherein the serotonin reuptake inhibitor or the compound which causes an elevation in the level of extracellular serotonin is used in the treatment of depression.

6. The use of any one of claims 1 to 5 wherein the serotonin reuptake inhibitor is selected from citalopram, escitalopram, fluoxetine, sertraline, paroxetine, fluvoxamine, venlafaxine, duloxetine, dapoxetine, nefazodone, imipramin, femoxetine and clomipramine or a pharmaceutically acceptable salt of any of these compounds.

7. The use of any one of claims 1 to 6 wherein the serotonin reuptake inhibitor is a selective serotonin reuptake inhibitor.

8. The use of any one of claims 1 to 7 wherein gaboxadol is in the form of an acid addition salt, or a zwitter ion hydrate or zwitter ion anhydrate.

9. The use of any one of claims 1 to 8 wherein gaboxadol is in the form of a pharmaceutically acceptable acid addition salt selected from the hydrochloride or hydrobromide salt, or in the form of the zwitter ion monohydrate.

10. The use of any one of claims 1-9 wherein the pharmaceutical composition comprises gaboxadol in an amount ranging from about 0.1 to about 2.5 mg.

11. The use of any one of claims 1-9 wherein the pharmaceutical composition comprises gaboxadol in an amount ranging from about 2.5 mg to about 20 mg.

12. The use of any one of claims 1-11 wherein gaboxadol is in an oral dose form.

13. The use of any one of claims 1-12 wherein the serotonin reuptake inhibitor or the compound which causes an elevation in the level of extracellular serotonin is in an oral dose form.

14. The use of claim 12 wherein gaboxadol is in a solid oral dose form, such as tablets or capsules, or a liquid oral dose form.

15. The use of claim 13 wherein the serotonin reuptake inhibitor or the compound which causes an elevation in the level of extracellular serotonin is in a solid oral dose form, such as tablets or capsules, or a liquid oral dose form.

16. The use of any one of claims 1-15 wherein gaboxadol is crystalline.

17. The use of any one of claims 1-16 wherein the pharmaceutical composition prepared is adapted for simultaneous administration of gaboxadol and the serotonin reuptake inhibitor.

18. The use of any one of claims 1-17 wherein gaboxadol and the serotonin reuptake inhibitor are contained in the same unit dosage form.

19. The use of any one of claims 1-18 wherein the pharmaceutical composition prepared is adapted for sequential administration of gaboxadol and the serotonin reuptake inhibitor.

20. A pharmaceutical composition comprising gaboxadol and a compound, which is a serotonin reuptake inhibitor, or any other compound which causes an elevation in the level of extracellular serotonin, and optionally pharmaceutically acceptable carriers or diluents.

21. The pharmaceutical composition of claim 20 **characterised in that** the serotonin uptake inhibitor is selected from citalopram, escitalopram, fluoxetine, sertraline, paroxetine, fluvoxamine, venlafaxine, duloxetine, dapoxetine, nefazodone, imipramin, femoxetine and clomipramine or a pharmaceutically acceptable salt of any of these compounds.

22. The pharmaceutical composition of any one of claims 20-21 which is adapted for simultaneous administration of gaboxadol and the serotonin reuptake inhibitor.

23. The pharmaceutical composition of any one of claims 20-22 wherein gaboxadol and the serotonin reuptake inhibitor are contained in the same unit dosage form.

24. The pharmaceutical composition of any one of claims 20-23 wherein gaboxadol is in the form of an acid addition salt, or a zwitter ion hydrate or zwitter ion anhydrate.

25. The pharmaceutical composition of any one of claims 20-24 wherein gaboxadol is in the form of a pharmaceutically acceptable acid addition salt selected from the hydrochloride or hydrobromide salt, or in the form of the zwitter ion monohydrate.

26. The pharmaceutical composition of any one of claims 20-25 wherein gaboxadol is present in an amount ranging from about 0.1 to about 2.5 mg.

27. The pharmaceutical composition of any one of claims 20-25 wherein gaboxadol is present in an amount ranging from about 2.5 mg to about 20 mg.

28. The pharmaceutical composition of any one of claims 20-27 wherein gaboxadol is in an oral dose form, such as a solid oral dose form, such as tablets or capsules, or a liquid oral dose form.

29. The pharmaceutical composition of any one of claims 20-28 wherein the serotonin reuptake inhibitor is in an oral dose form, such as a solid oral dose form, such as tablets or capsules, or a liquid oral dose form.

30. The pharmaceutical composition of any one of claims 20-29 wherein gaboxadol is crystalline.
